# EUROPEAN PATENT APPLICATION

(11) **EP 2 574 953 A1**
(43) Date of publication of application: **03.04.2013**
(21) Application number: 11462016.4
(22) Date of filing: 30.09.2011
(51) Int. Cl.: G01S 5/02, G06F 19/00, A61B 5/11, A61B 5/145

(54) **Method for providing remote health monitoring data and associated system**

(71) Applicant: General Electric Company, Schenectady, NY 12345 (US)
(72) Inventor: Dévényi, Csaba, 1034 Budapest (HU); Kósa, István, 8200 Veszprém (HU); Lohner, Roland, 1113 Budapest (HU); Végsö, Balázs, 8200 Veszprém (HU)
(74) Representative: Mak, Andras

(57) **Abstract**

A method and a system is proposed for providing remote health monitoring data of an individual to be used in a health monitoring system. The method comprises performing a measurement of a vital sign , receiving and storing the data representing the vital sign, and providing information on the a condition of the individual to be monitored for visual display. The method also comprises the steps of performing continuous measurement of a behavioral data of the individual to be monitored in addition to the measurement of the vital sign, and validating the data representing the vital sign in dependence of the behavioral data in order to increase reliability of the measurement of the vital signs.

## Description

### FIELD OF THE INVENTION

The invention relates generally to remote health monitoring, and in particular it relates to a method for providing remote health monitoring data in a reliable way. The invention further relates to a system for providing remote health monitoring data.

### BACKGROUND OF THE INVENTION

Health monitoring of individuals, especially of elderly people in their homes is getting more and more important as hospitals are often overcrowded, too far or too expensive on a longer time basis. Many attempts have been made in the past in order to facilitate remote health care of elderly people, and to provide methods and systems for providing health monitoring data for care taking persons at different levels from medical specialists, through health care personnel to family members. On the basis of the collected information some kind of diagnosis can be made by medical staff repeatedly, medication instructions can be changed if necessary or simply a signal may be given to family members when to call or visit an observed old-aged relative. In each case it is essential to collect reliable information which is not always possible with the currently used systems working in the homes of the individuals.

The patients are usually elder people, usually having a chronic condition, sometimes neurological diseases, which makes their actions unpredictable. The measurements in most of the cases are performed by the patients, without any professional support, so it's hard to guarantee the proper execution of the measurement procedure.

There are many home monitoring systems available on the market. In most of them it's possible for the patient to perform various measurements, and send the result of them to the physicians for review. The medical professionals supervising the monitoring of the patients make their decisions based on these measurement results, thus the quality and reliability of this information is essential.

US Patent no 7,684,999 discloses a user-based monitoring system including a remote user-based subsystem with at least one display and at least two microprocessor-based units in communication with each other. The subsystem is configured to facilitate collection of user-related data. The system also has at least one central server remotely located from and configured for two-way communication with the user-based subsystem so that it can receive and deliver signal communications to and from the user-based subsystem. The system also has at least one authorized user computer remotely located from, and configured for two-way signal communication with, the central server to receive user-related data collected by a remote user-based subsystem and allow an authorized user to communicate with the central server. The system is suited, amongst others, for monitoring remotely the health of a system user.

In the current commercial systems the measurements are performed by the patients, and it's up to the supervising medical professional to decide if the measured data is reliable.

Some systems, like Intel's Health Guide performs some validation of the data, but only based on the measurement result itself, e.g. if the measured blood pressure was too high, the patient is asked if they took their proper medication, or if they performed some intensive activity before the measurement.

Due to the disadvantages of the prior art systems and methods there is a continuous need for providing a method and system which makes it possible to improve reliability or to validate the result of measurements carried out by elderly people or patients suffering from different diseases, themselves.

### SUMMARY OF THE INVENTION

In one embodiment, a method is proposed for providing remote health monitoring data of an individual to be used in a health monitoring system, the method comprising the following steps:
- performing a measurement of a vital sign of the individual to be monitored,
- receiving and storing data representing the vital sign obtained in result of the measurement,
- providing information on a condition of the individual to be monitored for visual display.. The method further comprises the steps of:
- performing a continuous measurement of a behavioral data of the individual to be monitored in addition to the measurement of the vital sign, and
- validating the data representing the vital sign in dependence of the behavioral data in order to increase reliability of the measurement of the vital sign.

In another exemplary embodiment, a system is proposed for providing remote health monitoring data of an individual, the system comprising
- a plurality of subsystems at a location of the individual to be monitored, the plurality of subsystems comprising a subsystem control unit and at least one measuring unit for measuring a vital sign of the individual to be monitored, with at least a part of the at least one measuring unit in communication with the subsystem control unit,
- a central data server station in communication with the plurality of subsystems, and
- a monitoring side terminal in communication with the central station for providing an information for visual display.

The subsystem control unit is connected to at least one measuring unit for performing a continuous measurement of a behavioral data of the individual to be monitored in addition to the measurement of the vital sign in order to increase reliability of the measurement of the vital sign.

The subsystem control unit is configured to store the results of the measurement of the vital sign and the continuous measurement of the behavioral data and to validate the data representing the vital sign in dependence of the behavioral data.

In a further embodiment of the invention an improvement of the system is proposed, in which
- the subsystem control unit is connected with at least one measuring unit for performing continuous measurement of behavioral data of the individuals to be monitored in addition to the distinct measurements of vital signs in order to increase reliability of the measurement of the vital signs, and
- the central data collecting and processing station is configured to receive and store the results of the distinct measurements of vital signs and the continuous measurement of behavioral data and to validate the data representing the vital signs in dependence of the behavioral data.

Further advantageous embodiments of the invention are provided in the depending claims.

The proposed method and system for providing remote health monitoring data makes it possible to improve reliability or to validate the result of measurement of vital signs, such as blood pressure, blood glucose, ECG, weight, etc. carried out by elderly people or patients suffering from different diseases, themselves at any location distant from medical assistance, such as in a home environment.

Due to this improvement, the health care professionals and other car giving personnel can use more reliable data in assessing the current health state of an individual without personal consultation or clinical examination which is faster, less expensive and therefore more effective.

### BRIEF DESCRIPTION OF THE DRAWINGS

The invention will now be described in detail with reference to the accompanying drawing, in which
Fig. 1 is a schematic bock diagram of an embodiment of a monitoring subsystem,
Fig. 2 is a schematic bock diagram of an embodiment of an extended system for performing remote monitoring,
Fig. 3 is a schematic diagram of an embodiment of a behavioral monitoring system in the home environment
Fig. 4 is a schematic flow diagram of an embodiment of a method for performing vital sign measurements,
Fig. 5 is a schematic flow diagram of another embodiment of a method for performing vital sign measurements,
Fig. 6 is a schematic flow diagram of an embodiment of a method for performing verified vital sign measurements, and
Fig. 7 is a schematic flow diagram of another embodiment of a method for performing verified vital sign measurements.

### DESCRIPTION OF THE EMBODIMENTS

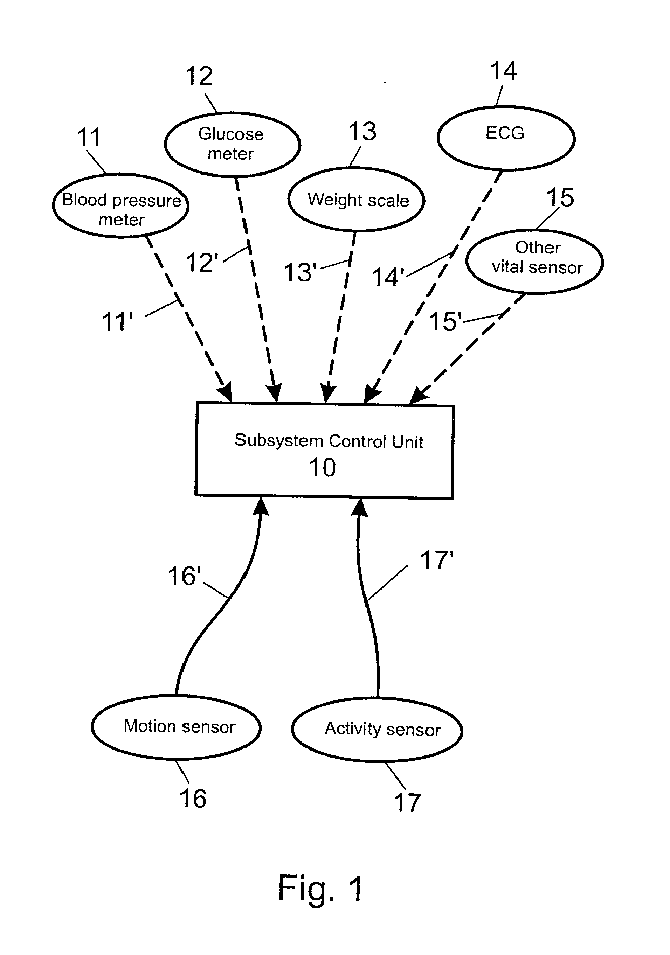

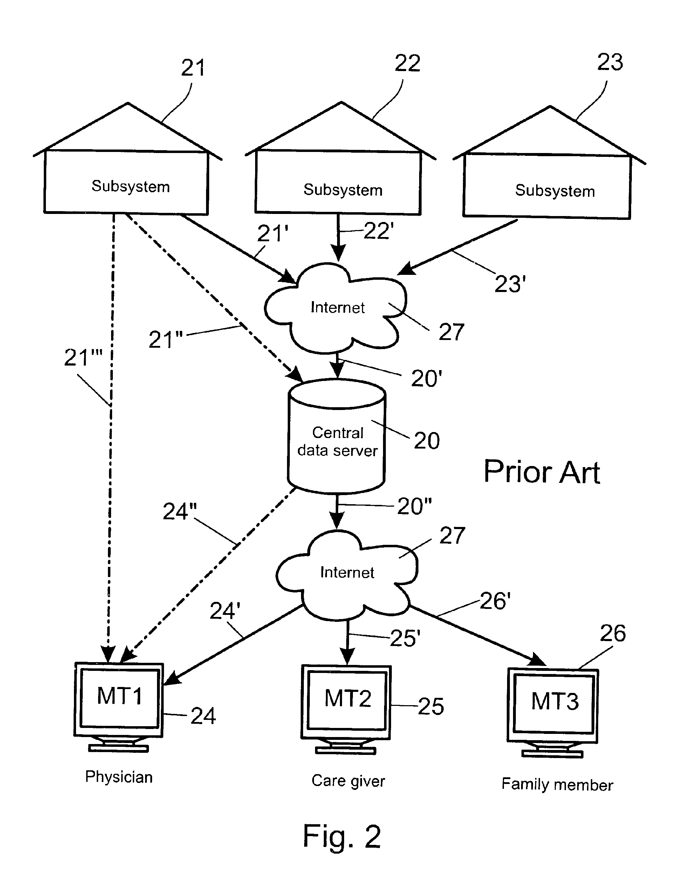

Referring first to Fig. 1, a schematic block diagram of one embodiment of a subsystem of a monitoring system is shown. The monitoring system will be explained in more detail on the basis of Fig. 2. The subsystem is provided with a subsystem control unit 10 (also called home hub) and at least one measuring unit 11 to 15 for measuring vital signs of the individuals to be monitored. The measuring units 11 to 15 for measuring vital signs have a communication link to the subsystem control unit 10 as indicated by the dotted lines 11' to 15'. These communication links 11' to 15' may be accomplished by wired communication links or by wireless communication links. The measuring units 11-15 for measuring vital signs of the individuals to be monitored may include for example but not exclusively, a blood pressure meter 11 for measuring the blood pressure, a glucometer 12 for measuring the blood glucose, weight scale 13 for determining the weight, an ECG monitor 14 for providing ECG data and other vital sign sensors 15 with the interaction of the monitored person. A part or all of these measuring units 11-15 may have a wireless communication link to the subsystem control unit 10 as indicated by the dotted lines 11' to 15'. The wireless communication may be performed by using a radio communication according to the Bluetooth, Zigbee, Wifi or other standardized or vendor specific proprietary specifications. Some of the devices that are outside the wireless communication range or do not have a wireless communication capability, may be connected to the subsystem control unit 10 by a communication wire, such as a USB cable or the like. The vital sign measuring devices need not to be specialized devices, any measurement device available off-the shelf may be suitable, even those which do not have any connection possibility. In this case, the home hub 10 used in the system must have a manual input capability, to enable the user to input the measuring result obtained from such a measurement device, e.g. a bathroom weight scale 13. Using such a device , the individual will read the result from the device and input (type) the value(s) using an input device (touchscreen, keyboard or similar) of the home hub. Such a subsystem for assisting elderly people or patients in carrying out vital sign measurement on their own, are known in the prior art such as disclosed in US Patent no 7,684,999.

One embodiment of the subsystem control unit 10 is also provided with at least one measuring unit 16 and 17 for performing continuous measurement of behavioral data of the individuals to be monitored in addition to the distinct measurements of vital signs in order to increase reliability of the measurement of the vital signs without any substantial interaction of the monitored person. In this case, the subsystem control unit 10 is configured to receive and store the results of the distinct measurements of vital signs and of the continuous measurement of behavioral data. The subsystem control unit 10 uses the collected behavioral data to validate the data representing the vital signs of an individual to be monitored remotely. The measuring units 16 and 17 for performing continuous measurement of behavioral data of the individuals to be monitored may include for example but not exclusively at least one fix mounted motion sensor 16 for determining the motion and/or location of the individual in a selected area, and/or at least one body-worn sensor 17 for sensing the activity such as speed and/or acceleration of the motion of a selected body part of the individual for determining the motion activity of the individual. The fix mounted motion sensors 16 may communicate with the subsystem control unit 10 using either a wireless or a wired communication link 16'. In case of the body-worn sensors 17 however it may be more advantageous if they are communicating with the subsystem control unit 10 using either through a wireless communication link 17' so as not to restrict the wearer in his or her movement in any way. As a wired or wireless communication link 16' and 17', the same or similar communication link may be selected as the ones used for the measuring unit 11 - 15 for measuring vital signs.

The fix mounted sensors 16 may be for example motion detectors or contact sensors mounted on walls or other pieces of furniture or equipments of the living area of the individuals to be monitored. These sensors 16 are not in direct contact with the monitored person. The contact sensors (not separately shown) can have different function in a monitored area. Typical installation points are the front door of the house/apartement, doors which might be useful to know if open or closed (e.g. bathroom door), doors of household equipment (e.g. door of the fridge) and critical places healthcare related monitoring(e.g. if the person keeps all the medication in a closed drawer or box, the door of this holder). Motion sensors 16, such as passive infrared sensors (PIR sensors) NO can measure infrared light radiating from objects in its field of view. Actual motion is detected when an infrared source NO with one temperature changes its position in front of an infrared source with another temperature, so in our case when a human passes the sensor's field of view in the monitored are. If there's a higher amount of motion then a pre-defined threshold, the sensor 16 sends a signal to the subsystem control unit 10.

The body-worn sensor 17 may be for example an activity sensor such as a speed or acceleration sensor fixed to a part of the body, preferably to a hand or arm or to a leg or foot of the wearer. These sensors 17 are in direct contact with the monitored person. The body-worn sensor or actigraph 17 as it is generally called, is a body-worn equipment, most often worn on the wrist, like a wrist watch. The unit 17 continually records the movement of the equipment itself, therefore the movement of the patient's wrist. This data can be used to calculate the motion of the monitored person (overall activity, step count, etc.).

The Actigraph unit 17 generally consists of the following:
- Accelerometers, along 3 axis, to detect the acceleration of the unit
- Memory, to store the recorded data, until it's uploaded to a permanent storage
- An interface , in our case Bluetooth unit, to communicate with the homehub , sending the collected data
- battery

Also a docking unit is part of the equipment. The docking unit has power supply. The docking unit has two purposes:
- charges the battery of the actigraph, when it is placed in the docking unit
- the actigraph can detect, if it is placed in the docking unit , and based on its settings, it can initiate data transfer.

The normal use of the actigraph 17 is that the monitored person wears the unit during his wake hours, putting it on when he wakes up, and putting it into its docking unit before going to sleep at night. Therefore during the wake hours the actigraph 17 collects all motion performed by the monitored person, while during his sleep it charges the battery of the unit and transfers all collected data to a data storing unit , such as a home hub 10 in our case.

A remote health monitoring system using the above subsystem is schematically shown in Fig. 2. The system comprises a plurality of subsystems 21, 22, 23 at the location of the individuals to be monitored distant from medical assistance, such as in a home environment. In the simplest case a subsystem, such as subsystem 21 may be connected to a monitoring terminal 24, 25, 26, such as monitoring terminal 24 via a communication channel 21'. The communication channel 21' may be either a radio or a cable communication channel. In this case a monitoring person may have only access to one individual to be monitored at a time. Each change of the monitored person would require a reconnection to another communication channel. This problem can be solved by using a central communication and data server 20, which is capable of communicating with the subsystems 21, 22, 23 via data communication channels, through a cable or an air interface. Preferably, the system also comprises a number of monitoring terminals 24, 25, 26 which are capable of communicating with the central server station 20 in order to provide information for visual display to the monitoring persons, such as health care professionals and/or care giving personnel and/or authorized family members. Each group of the monitoring persons has a predetermined access right category to access monitoring information provided by the subsystems 21, 22, 23 and the central server unit 20. The health care professionals may for example be authorized to functionalities such as browsing patient data and setting up the monitoring parameters for the individual patient. The caregiver personnel may be authorized to browsing patient data and preparing different reports based on it. The family members may be authorized to have access to their respective relative in order to have information about his or her health condition. The monitoring terminals 24, 25, 26 may be connected to the central server 20 through either a radio communication channel or a cable communication channel, or a combination of a radio communication channel and a cable communication channel 24', 25', 26', such as the Internet 27 . The use of the Internet as a communication channel makes it possible to set up the elements of the remote health monitoring system at any location of the world without any limitation. Therefore in a most flexible configuration, the elements of the system , e.g. the subsystems 21, 22, 23 are connected through communication links 21', 22', 23', the central server unit 20 through communication links 20' and 20", and the monitoring terminals 24, 25, 26 through communication links 24', 25', 26' to the Internet 27. In a general configuration of Fig. 2 the central server station 20 receives and stores all the data from the connected home hubs (subsystem control units) and provides access to information about the health condition of all individuals included in the system to the authorized monitoring persons.

In this configuration of the system for determining health condition parameters of individuals remotely, the subsystem control unit 10 is connected to at least one measuring unit 16, 17 for performing continuous measurement of behavioral data of the individuals to be monitored in addition to the distinct measurements of vital signs in order to increase reliability of the measurement of the vital signs (see Fig. 1). The subsystem control unit 10 further may be configured to receive and store the results of the distinct measurements of vital signs and the continuous measurement of behavioral data and to /validate the data representing the vital signs in dependence of the behavioral data. In this case each of the subsystem control unit 10 has to be programmed so that they are capable of not only collecting the measurement results but also /validate them in dependence of the collected behavioral data. Validation of the data representing the vital signs means a decision whether the vital sign measuring results are acceptable and reliable for assessing the health state of the individuals to be monitored.

In an alternative embodiment, the central data server station 20 is configured to receive and store the results of the distinct measurements of vital signs and the continuous measurement of behavioral data and to validate the data representing the vital signs in dependence of the behavioral data. In this case the subsystem control units 10 need not be provided with a special validation program and only the central data server unit 20 has to be programmed so that it is capable of collecting the measurement results but also validate them in dependence of the collected behavioral data. In both cases the monitoring persons (health care specialist, caregiver personnel, family members) having right to access the monitoring data may retrieve the validated and therefore reliable monitoring data through a monitoring terminal 24, 25, 26 as shown in Fig. 2 from the central data server 20. In the example shown in Fig. 2, the monitoring terminal 24 is a terminal for the health care specialists, the monitoring terminal 25 is a terminal for the caregiver personnel and the monitoring terminal 26 is a terminal for the family members. Although there are only three monitoring terminals 24, 25, 26 shown in the drawing, a person skilled in the art will appreciate, that any other number of terminals may be selected arbitrarily according to the need of a specific application.

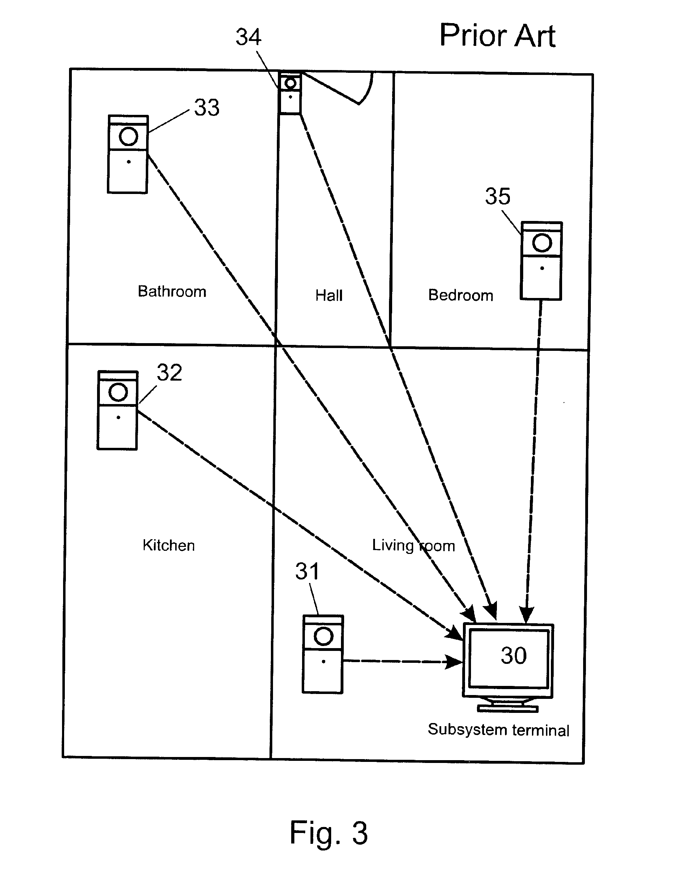

Fig. 3 shows an exemplary arrangement of the elements of a behavioral monitoring subsystem at a location of an individual to be monitored, which is preferably at a distant location from the caregivers and/or the health care specialists and/or the family members. This location may be typically in the home of the individuals to be monitored, who are generally elderly people or patients released from hospital. In this arrangement the subsystem control unit 10 is shown as a subsystem terminal 30 with a number of fix mounted sensors 16 (contact sensors and motion sensors) that are connected to the subsystem terminal 30 through a radio or cable communication channel. The contact sensors (not shown) are simple devices, consisting of two matching parts. A contact sensor generally can detect, if these two parts are in contact, or separated, and when a change happens in its state, it sends a signal to the subsystem control unit 10. So e.g. if one part of this sensor is mounted to a door, and the other is to the frame of the door, the sensor can detect if the door is open or closed. Such sensors may be mounted on matching surfaces being relatively moved with respect to each other, such as a door of a room, a door of a piece of furniture or the door of an equipment, such as a refrigerator. In this configuration, each room (e.g. bathroom, hall, bedroom, kitchen and living room) has an own motion detector 31 to 35, which may be preferably wall mounted. This way the system can provide information in which room the monitored person is at a moment. If there are rooms, where there are well separated areas with different, meaningful purpose (living room and kitchen in one common area), more than one motion sensor can be mounted in one room, setup in a way to cover different areas with their field of view. These sensors 31 to 35 provide continuous measurement without any interaction of the individuals to be monitored. They are not in direct contact with the monitored person. The sensors 31 to 35 of the subsystem 21, 22, 23 thus configured provide continuous information regarding to the location of the individual being monitored and further information on closing or opening a door of a room, a piece of furniture or an equipment, that allows conclusions on the current activity of the individuals being monitored.

The body-worn activity sensors 17 of Fig. 1 that are mounted on a body part of the individuals (not shown) are also connected to the subsystem control unit 10, preferably though a wireless communication channel 17'. These sensors 17 provide continuous information on the activity - such as speed or acceleration of selected body parts - of the individuals to be monitored. The wireless communication link 17' between the body-worn sensors (also called actigraph) 17and the subsystem control unit 10 may be for example a communication link according to the Bluetooth, Zigbee, Wifi or other standardized specifications. In some cases Zigbee may be preferred because of its flexibility and low power consumption.

The subsystem control 10 unit or home hub may include a general computer provided with a suitable cable and/or radio interface units, a storage unit for storing the measured data, input and output devices for communication with the individuals to be monitored. Such input devices may include for example but not exclusively a keyboard, a pointing device, such as a mouse, a touchpad, or a touch-screen, etc. The output devices may include for example but not exclusively a monitor, preferably a flat screen monitor, a printer, an audio output device, such as a loudspeaker, etc. The interface units typically form integral part of the subsystem control unit, but it is also possible to connect external interface units to the subsystem control unit, e.g. via a USB connector. Such a subsystem control unit is known per se (such as Intel Healthguide) and needs not to be explained further.

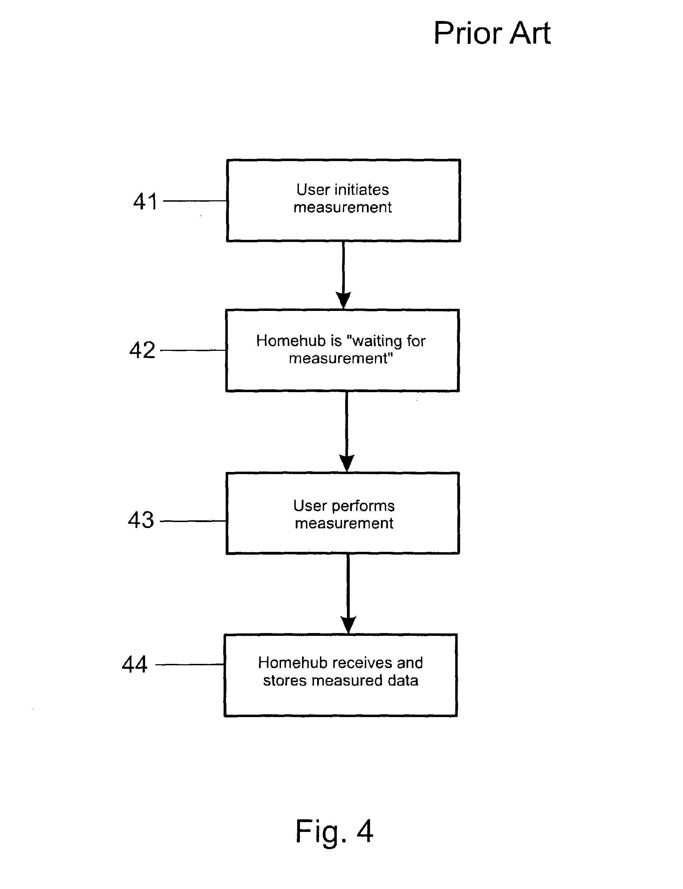

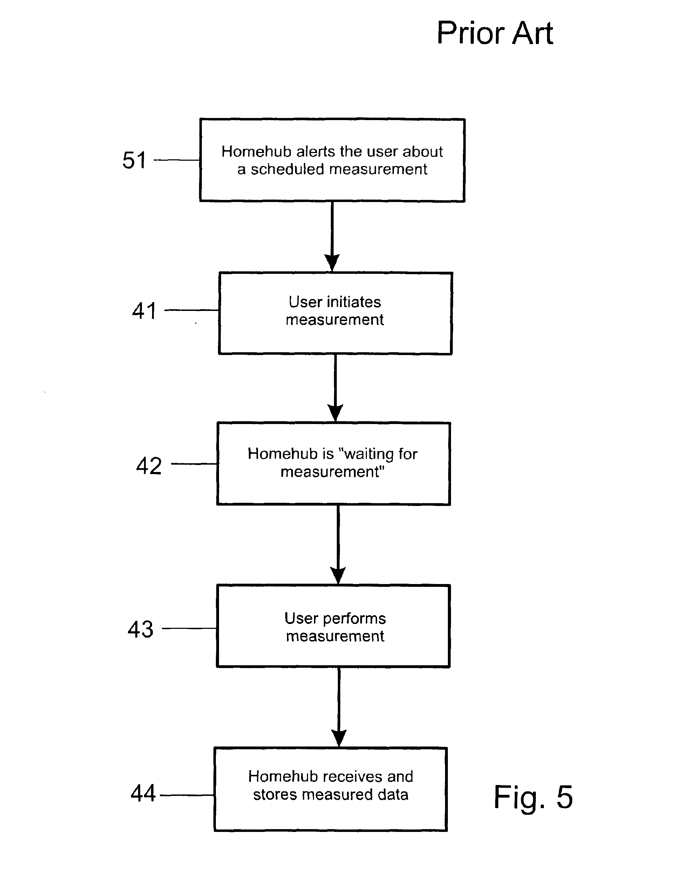

There are two scenarios, when the vital sign measurements are performed by the user, using the available measurement devices. According to the first scenario, the measurement can be initiated by the users themselves, without any instruction from the system itself. According to a second scenario, if the system contains scheduling for the measurements, the initiating of a measurement can be requested by the system itself. The concept described here applies to both cases, so we don't have to separately discuss them, it does not matter why a measurement has been initiated. These two scenarios are depicted in Figs. 4 and 5 as a schematic flow diagram. In a first step 51 (Fig. 5) the subsystem here not visible requires the individual to be monitored to initiate a scheduled measurement. This may be the case when a number of individuals have to carry out the same measurement at substantially the same time, e.g. measurement of blood pressure, temperature, weight in the morning and in the evening. After the first step discussed above all the following steps (of Figs. 4 and 5) are identical and therefore identical reference signs have been used to indicate the identical steps. In step 41 the individual to be monitored initiates a measurement with a vital sign measuring device not shown here connected to the home hub (e.g by pressing a button with the label "Measure my blood pressure"). In response to this, the home hub switches to a "waiting for incoming measurement" mode in a following step 42. In a next step 43, the user performs the selected measurement with the selected device (e.g. measures his blood pressure using the blood pressure meter). In step 44 the home hub receives and stores the measured data, e.g. the result of the vital sign measurement. To this end the measurement device initiates the connection to the home hub, and sends the data just measured by the individual. The home hub acknowledges the received measurement result, and informs the user whether the measurement was successful.

Depending on the system architecture, the measurement data is either sent to the central data server's database immediately, or can be stored in the homehub , if it is possible temporarily, and sent (e.g in daily packages) to the central data server unit. On the basis of the collected vital sign measurement data, it is possible to provide information on the actual condition of the individuals to be monitored to health care specialists and/or care giving personnel and/or authorized family members in form of a visual display.

An embodiment of the method however comprises the further steps of
- performing continuous measurement of behavioral data of the individuals to be monitored in addition to the distinct measurements of vital signs, and
- validating the data representing the vital signs in dependence of the behavioral data

in order to increase reliability of the measurement of the vital signs.

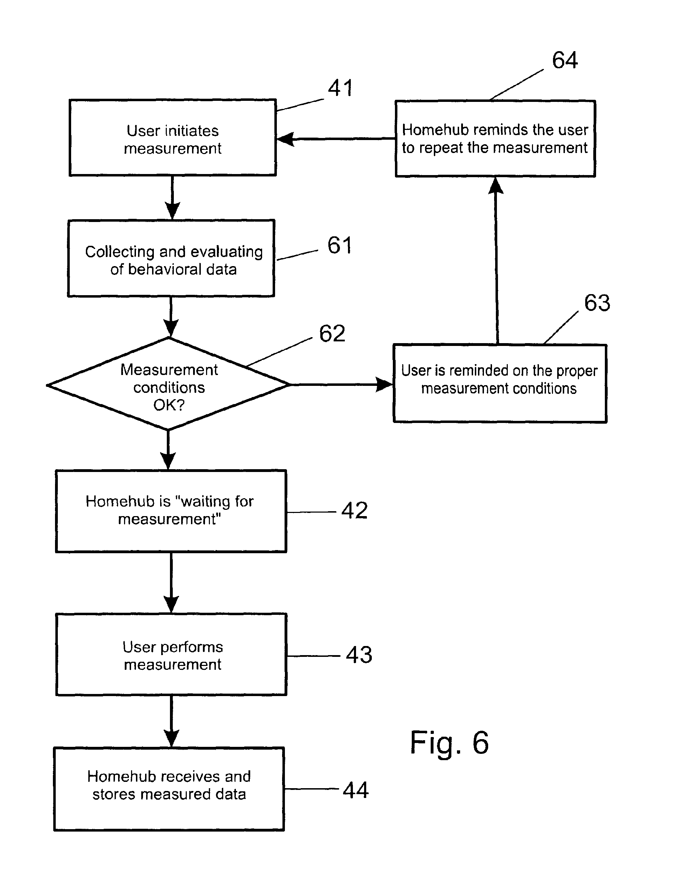

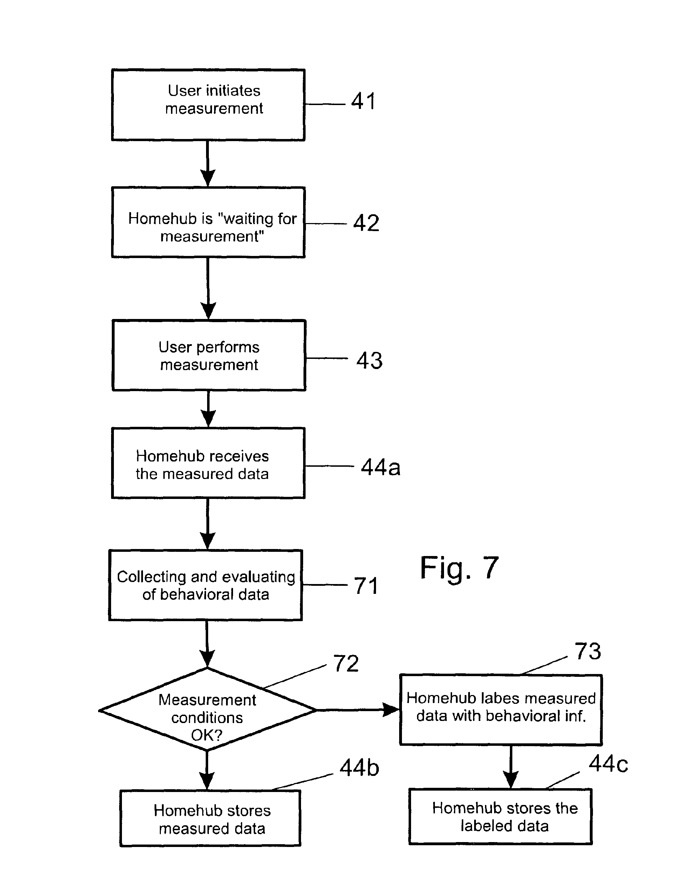

Examples of the inventive concept with respect to the above features will be explained with reference to Figs. 6 and 7. There are two approaches to validate the measurement conditions, before and after the measurement: validating the measurement conditions before the measurement and validating the proper conditions after the measurement. Validating the measurement conditions before the measurement eliminates unnecessary measurements, and ensures more valid data measurements. It requires real-time data processing, and immediate response to the user. It means step 62 is an immediate decision, so if we continue to 63, right after the user pressed the "Measure my blood pressure" button, a message appears on the screen saying "You've had significant activity in the last few minutes, please relax and perform the measurement in 3 minutes". Validating the proper conditions after the measurement can be performed anytime, even days later than the actual measurement, on any device. Of course, if the measurement conditions were not proper, it is not possible to repeat the measurement, and gather proper data from that moment.

The procedure to perform a measurement after validating and/or creating the proper conditions of the measurement is shown in Fig. 6. As it can be clearly seen in Fig. 6, the steps 41 to 44 already discussed in connection with Fig. 4 are carried out in the same order. However between steps 41 and 42 the steps for establishing the proper conditions for a measurement are inserted. After the user has initiated a measurement in step 41, the home hub collects and processes the behavioral data in order to make sure that the measurement condition are appropriate, in step 61. In step 62 the home hub decides whether the proper conditions for the selected measurement are met or not. If yes, the procedure continues according to a normal measurement procedure as already explained with reference to Fig. 4. If, however the proper conditions for the selected measurement are not met, the home hub informs the user that the conditions are not appropriate for the measurement. Further the home hub informs the user of the required conditions and the actions and/or time required to achieve the required conditions, in step 63. After a predetermined time, in step 64 when the required conditions are expected to be met, the user is reminded to repeat the selected measurement. This leads therefore back to step 41 of initiating a measurement by the user (individual).

The procedure to perform a measurement before validating or establishing the proper conditions of the measurement is shown in Fig. 7. As it can be clearly seen in Fig. 7, the steps 41 to 44 already discussed in connection with Fig. 4, are carried out in the same order. However in step 44 the measured vital sign data are only received and not stored in order to be transferred to the central data server and for being retrieved by the monitoring persons. After step 44 the steps for examining and validating the proper conditions for a measurement are introduced. This includes step 71, in which the home hub collects and processes the behavioral data in order to make sure that the conditions at the time of the measurement were appropriate. In step 72 the home hub decides whether the proper conditions for the selected measurement were met or not. If yes, the procedure continues according to a normal measurement procedure, that is the validated measurement result is stored in step 44b. If, however the proper conditions for the selected measurement are not met, the home hub labels the measurement data in step 73 and stores the measurement data with the additional data labels describing the measurement conditions (including all information useful for the person evaluating the results), in step 44c.

As explained above, the invention provides a method and a system for carrying out the method based on a known vital sign measurement method, with the addition, that behavioral data are collected from the monitored persons, which is used to make sure the measurement has been made in the proper condition for the measurement and the person and all measurements according to the invention are validated or labeled with a validation information. This has the following main advantages compared to the traditional methods. The method of the invention can enhance data quality by changing the monitored person's behavior in order to establish the proper measurement conditions. Even if - in an alternative - the measurement condition is not changed, additional behavioral information (labeled data) makes collected data more useful for the person evaluating it.

The following examples are intended to provide a better understanding of the proper measurement conditions:
Example 1: weight measurement
   Weight can change significantly depending if it was measured before or after eating, drinking and bathroom visits. The most appropriate moment to measure weight for a monitored person is in the morning, after the first bathroom visit and before eating, drinking. Using the mounted behavioral monitoring system, it is possible to detect if the measurement has been taken with these conditions. In this case, reminding the monitored person to the proper conditions means that we have detected that no bathroom visits happened yet after waking up, and we ask them to perform the measurement after visiting the bathroom. (see Fig. 6)
Example 2: blood glucose measurement
   Blood glucose levels are absolutely dependent on the food intake. Using the mounted behavioral monitoring system, with sensors applied to possible food sources, fridge, closet, it's possible to detect if the monitored person potentially had food intake, which does not comply with the prescribed measurement conditions.
   In case of blood glucose measurement, establishing proper measurement conditions is usually not possible, so adding the food intake information to the data is performed (see Fig.7)
Example 3: blood glucose measurement
   Blood pressure and ECG measurement results are dependent on the amount and intensity of the activity performed before the measurement. The activity data can come from the body worn sensors, providing a quite precise measurement on both activity and intensity, or the mounted sensors, providing data on excessive amount of movement in the living area. Also, if the mounted sensors show that the monitored person has just arrived home, it also requires a resting period before the measurement.
   In these cases creating a proper measurement condition is easy, the monitored person just needs to rest for a few minutes. The home hub can even remind the monitored person if the resting period was enough, and the measurement can be performed (see Fig. 6).

This written description uses examples to disclose the invention, including the best mode, and also to enable any person skilled in the art to make and use the invention. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

## Claims

1. Method for providing remote health monitoring data of an individual to be used in a health monitoring system comprising the steps of
- performing a measurement of a vital signs of the individual to be monitored,
- receiving and storing data representing the vital sign obtained in result of the measurement,
- providing information on a condition of the individual to be monitored for visual display,
**characterized in** the further steps of
- performing a continuous measurement of a behavioral data of the individual to be monitored in addition to the measurement of the vital sign, and
- validating the data representing the vital sign in dependence of the behavioral data.

2. The method of claim 1, in which the vital sign measurement includes the measurement carried out by a vital sign measurement device.

3. The method of claim 1, in which the step of performing continuous measurement of behavioral data of the individuals to be monitored comprises any of the following steps:
- determining the motion and/or location of the individual in a larger area by fix mounted motion sensors, and
- determining the motion activity of the individual by body-worn sensors sensing the speed and/or acceleration of the motion of a selected body part of the individual.

4. The method of claim 3, in which the determined behavioral data is compared to a reference or required behavioral data of the individual for each time of measurement of the vital signs and in case of divergence from the reference or required behavioral data, a message (audio and/or visual) is generated and communicated to the individual to be monitored.

5. The method of claim 4, in which the reference or required behavioral data is stored as a predetermined empirical value for all types of vital sign measurements.

6. The method of claim 4, in which the message reminds the individual of the required behavioral circumstances of a specific measurement and suggests to carry out the measurement a predetermined time after establishing the required behavioral circumstances of the measurement.

7. The method of claim 6, in which a message is generated that reminds the individual of expiry of the predetermined time.

8. The method of claim 3, in which the determined behavioral data is compared to a reference or required behavioral data of the individual for each time of measurement of the vital signs and in case of divergence from the reference or required behavioral data, the result of the measurement of the vital signs is labeled with the circumstances of measurement, and handled as not trustworthy data.

9. System for providing remote health monitoring data of an individual comprising
- a plurality of subsystems (21 ... 23) at a location of the individual to be monitored, the plurality of subsystems comprising a subsystem control unit (10) and at least one measuring unit (11 - 15) for measuring a vital sign of the individual to be monitored, with at least a part of the at least one measuring unit in communication with the subsystem control unit (10),
- a central data server station (20) in communication with the plurality of subsystems (21 - 23), and
- a monitoring side terminal (24, 25, 25) in communication with the central station (10) for providing an information for visual display,
**characterized in that**,
- the subsystem control unit (10) is connected to at least one measuring unit (16, 17) for performing a continuous measurement of a behavioral data of the individual to be monitored in addition to the measurement of the vital sign in order to increase reliability of the measurement of the vital sign, and
- the subsystem control unit (10) is configured to receive and store the results of the measurement of the vital sign and the continuous measurement of the behavioral data and to validate the data representing the vital signs in dependence of the behavioral data.

10. The system of claim 9, in which the measuring units (11 - 15) for measuring vital sign of the of individual to be monitored are selected from the group comprising measuring units for measuring the weight, temperature, blood pressure, blood glucose and ECG or any other vital sign of the individual to be monitored.

11. The system of claim 9, in which the measuring unit for performing continuous measurement of behavioral data of the individuals to be monitored comprises any of the following:
- at least one fix mounted motion sensor (16) for determining the motion and/or location of the individual in a selected area, and
- at least one body-worn sensor (17) for sensing the activity such as speed and/or acceleration of the motion of a selected body part of the individual for determining the motion activity of the individual.

12. The system of claim 10, in which at least a part of the measuring units (11 - 15) for measuring vital signs of the individuals to be monitored is connected through wires to the subsystem control unit (10) of the .

13. The system of claim 10, in which at least a part of the measuring units (11 - 15) for measuring vital signs and the measuring units (16, 17) for performing continuous measurement of behavioral data of the individuals to be monitored are connected through wireless connection to the subsystem control unit (10) of the.

14. System for providing remote health monitoring data of an individual comprising
- a plurality of subsystems (21 - 23) at a location of the individual to be monitored, the plurality of subsystems comprising a subsystem control unit (10) and at least one measuring unit (11 - 15) for measuring a vital sign of the individual to be monitored, with at least a part of the at least one measuring unit in communication with the subsystem control unit,
- a central data server station (20) in communication with the subsystems (21 - 23), and
- a monitoring side terminal (24, 25, 26) in communication with the central station (20) for providing information for visual display,
**characterized in that**,
- the subsystem control unit (10) is connected with at least one measuring unit (16, 17) for performing a continuous measurement of a behavioral data of the individual to be monitored in addition to the measurements of the vital sign in order to increase reliability of the measurement of the vital sign, and
- the central data server unit (20) is configured to receive and store the results of the measurement of the vital sign and the continuous measurement of the behavioral data and to validate the data representing the vital signs in dependence of the behavioral data.

15. The system of claim 15, in which the measuring units (11 - 15) for measuring vital signs of the of individuals to be monitored are selected from the group comprising measuring units for measuring the weight, temperature, blood pressure, blood glucose and ECG or any other vital sign of the individuals to be monitored.

16. The system of claim 15, in which the measuring unit for performing continuous measurement of behavioral data of the individuals to be monitored comprises any of the following:
- at least one fix mounted motion sensor (16) for determining the motion and/or location of the individual in a selected area, and
- at least one body-worn sensor (17) for sensing the speed and/or acceleration of the motion of a selected body part of the individual for determining the motion activity of the individual.

## Amended claims

### Amended claims in accordance with Rule 137(2) EPC.

**1.** A computer implemented method for providing remote health monitoring data of an individual to be used in a health monitoring system comprising the steps of
- performing a measurement of a vital sign of the individual to be monitored,
- receiving and storing data representing the vital sign obtained in result of the measurement,
- performing a continuous measurement of a behavioral data of the individual to be monitored in addition to the measurement of the vital sign,
**characterized in** the further steps of
- providing reference or required behavioral data as a predetermined value for all types of vital sign measurements,
- comparing the behavioral data at the time of performing a vital sign measurement with the predetermined reference or required behavioral data and
- validating the data representing the vital sign in dependence of the result of the comparison.

**2.** The method of claim 1, in which the vital sign measurement includes the measurement carried out by a vital sign measurement device.

**3.** The method of claim 1, in which the step of performing continuous measurement of behavioral data of the individuals to be monitored comprises any of the following steps:
- determining the motion and/or location of the individual in a larger area by fix mounted motion sensors, and
- determining the motion activity of the individual by body-worn sensors sensing the speed and/or acceleration of the motion of a selected body part of the individual.

**4.** The method of claim 1, in which in case of divergence of the determined behavioral data from the reference or required behavioral data, a message (audio and/or visual) is generated and communicated to the individual to be monitored.

**5.** The method of claim 4, in which the message reminds the individual of the required behavioral circumstances of a specific measurement and suggests to carry out the measurement a predetermined time after establishing the required behavioral circumstances of the measurement.

**6.** The method of claim 5, in which a message is generated that reminds the individual of expiry of the predetermined time.

**7.** The method of claim 1, in which in case of divergence of the determined behavioral data from the reference or required behavioral data, the result of the measurement of the vital signs is labeled with the circumstances of measurement, and handled as not trustworthy data.

**8.** A computer implemented system for providing remote health monitoring data of an individual comprising
- a plurality of subsystems (21 ... 23) at a location of the individual to be monitored, the plurality of subsystems comprising a subsystem control unit (10) in form of a first computer unit and
- at least one measuring unit (11- 15) for measuring a vital sign of the individual to be monitored, with at least a part of the at least one measuring unit in communication with the subsystem control unit (10),
- at least one measuring unit (16, 17) in communication with the subsystem control unit (10) for performing a continuous measurement of a behavioral data of the individual to be monitored in addition to the measurement of the vital sign in order to increase reliability of the measurement of the vital sign,
- a central data server station (20) in form of a second computer unit in communication with the plurality of subsystems (21 - 23), and
- a monitoring side terminal (24, 25, 25) in communication with the central station (10) for providing an information for visual display, **characterized in that**,
- one of the computer units (10, 20) is configured to store reference or required behavioral data as a predetermined value for all types of vital sign measurements,
- to compare the behavioral data at the time of performing a vital sign measurement with the predetermined reference or required behavioral data and to validate the data representing the vital signs in dependence of the result of the comparison.

**9.** The system of claim 8, in which the measuring units (11 - 15) for measuring vital sign of the of individual to be monitored are selected from the group comprising measuring units for measuring the weight, temperature, blood pressure, blood glucose and ECG or any other vital sign of the individual to be monitored.

**10.** The system of claim 8, in which the measuring unit for performing continuous measurement of behavioral data of the individuals to be monitored comprises any of the following:
- at least one fix mounted motion sensor (16) for determining the motion and/or location of the individual in a selected area, and
- at least one body-worn sensor (17) for sensing the activity such as speed and/or acceleration of the motion of a selected body part of the individual for determining the motion activity of the individual.

**11.** The system of claim 9, in which at least a part of the measuring units (11-15) for measuring vital signs of the individuals to be monitored is connected through wires to the subsystem control unit (10).

**12.** The system of claim 9, in which at least a part of the measuring units (11 - 15) for measuring vital signs and the measuring units (16, 17) for performing continuous measurement of behavioral data of the individuals to be monitored are connected through wireless connection to the subsystem control unit (10) .
